(19) Europäisches Patentamt / European Patent Office / Office européen des brevets

(11) **EP 3 563 087 B1**

(12) **FASCICULE DE BREVET EUROPEEN**

(45) Date de publication et mention de la délivrance du brevet:
**23.12.2020 Bulletin 2020/52**

(51) Int Cl.:
*F23D 3/24* *(2006.01)*  *A01M 1/20* *(2006.01)*
*A61L 9/03* *(2006.01)*  *F23D 3/40* *(2006.01)*

(21) Numéro de dépôt: **17828994.8**

(22) Date de dépôt: **21.12.2017**

(86) Numéro de dépôt international:
**PCT/FR2017/053759**

(87) Numéro de publication internationale:
**WO 2018/122501 (05.07.2018 Gazette 2018/27)**

(54) **BRÛLEUR À COMBUSTION CATALYTIQUE EN MATERIAU POREUX A PERFORMANCES DE FONCTIONNEMENT OPTIMISÉES ET FLACON EQUIPÉ D'UN TEL BRÛLEUR**

KATALYTISCHER VERBRENNER AUS EINEM PORÖSEN MATERIAL MIT OPTIMIERTER BETRIEBSLEISTUNGEN UND MIT SOLCH EINEM BRENNER AUSGESTATTETE FLASCHE

CATALYTIC COMBUSTION BURNER CONSISTING OF A POROUS MATERIAL WITH OPTIMISED OPERATING PERFORMANCES AND BOTTLE PROVIDED WITH SUCH A BURNER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.12.2016 FR 1663556**

(43) Date de publication de la demande:
**06.11.2019 Bulletin 2019/45**

(73) Titulaire: **Produits Berger**
**27520 Grand Bourgtheroulde (FR)**

(72) Inventeurs:
• **GERARD, Corinne**
 **27400 Incarville (FR)**

• **PAJOT, Laetitia**
 **87920 Condat sur Vienne (FR)**
• **CELLIER, Matthieu**
 **87510 Saint Jouvent (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**FR-A1- 2 779 509   FR-A1- 2 905 164**
**TW-A- 201 215 417   US-A1- 2008 014 539**

**Description**

La présente invention concerne de manière générale le domaine de la combustion catalytique, et plus précisément celui des brûleurs à combustion catalytique en matériau poreux. Ces brûleurs sont en particulier utilisés pour la diffusion de parfum et ou de substances actives, pour la destruction de molécules odorantes ou non, et/ou pour l'assainissement de l'air.

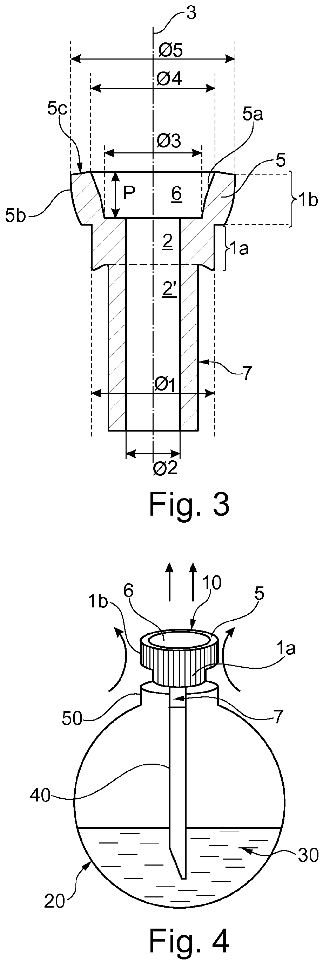

Un tel brûleur a par exemple été décrit dans le brevet FR2610390 au nom de la demanderesse. Il est notamment destiné à recevoir une mèche trempant dans un liquide combustible contenu dans un flacon à combustion catalytique, qui reçoit le brûleur au niveau de son goulot. Un tel brûleur (notamment représenté sur la figure 3) est réalisé en un matériau poreux, qui comprend un embout présentant à sa partie supérieure une cavité débouchant à l'extérieur et à sa partie inférieure, une cavité dans laquelle est engagée l'extrémité de la mèche. L'embout se prolonge à sa partie inférieure par un manchon. En fonctionnement, le liquide combustible amené par la mèche pénètre dans les pores du matériau poreux du brûleur. Une partie de ce liquide traverse la zone centrale du brûleur et y subit une vaporisation.

Un brûleur catalytique à corps poreux selon le préambule de l'objet de la revendication 1 est par ailleurs décrit par le brevet FR2905164.

Il est recommandé de remettre régulièrement du liquide combustible dans le flacon. Toutefois, l'expérience montre que de nombreux utilisateurs oublient d'arrêter le brûleur et le laisse fonctionner jusqu'à consommation complète du liquide combustible. Lorsqu'il n'y a plus de liquide combustible, la mèche sèche et son extrémité supérieure, emprisonnée dans la partie supérieure de la cavité interne du brûleur, au contact des parois chaudes de cette cavité, tend à brûler en l'absence d'oxygène et à charbonner avec production de particules libres de carbone qui viennent boucher les pores du brûleur. Il en est de même pour les dernières quantités utilisables de liquide combustible véhiculées par la mèche à un débit décroissant, qui ne traversent plus les parois du brûleur et dont la combustion incomplète libère des particules de carbone amorphe. Le brûleur perd ainsi son efficacité, devient inutilisable et doit être changé.

En outre, de nombreux utilisateurs souhaitent de plus en plus pouvoir utiliser en continu le flacon à combustion catalytique à partir d'un état de remplissage total, de manière à pouvoir l'utiliser plus longtemps. Cela implique que le manchon (ou fût) du brûleur doit être plus long, typiquement de l'ordre de 20 mm Toutefois, un tel brûleur à fût long non dopé en catalyseur sur sa partie supérieure n'est pas approprié pour fonctionner en présence d'un dispositif de climatisation (climatisation, ou ventilation, ou courant d'air). Par conséquent, les brûleurs à fût long certains sont refusés dans certains marchés (notamment en Asie ou aux Etats-Unis où le climat est chaud et humide) car associés à ce dysfonctionnement (alors que ce n'est pas toujours vrai, notamment en présence d'un catalyseur), pour lesquels il est habituel d'utiliser de tels dispositifs. Pour ces pays, le fût long est synonyme de dysfonctionnement.

Afin de pallier les inconvénients précités, la demanderesse a mis au point un brûleur à combustion catalytique en matériau poreux comprenant :

- un embout comportant :

  • une partie inférieure de diamètre extérieur $\phi_1$ et délimitant une première cavité de diamètre $\phi_2$, ladite cavité s'étendant le long d'un axe principal et étant adaptée à recevoir une mèche propre à imbiber l'embout d'une composition combustible, et
  • une partie supérieure présentant une paroi périphérique latérale comportant une face intérieure de forme essentiellement tronconique et délimitant une deuxième cavité de profondeur P et une face extérieure de forme essentiellement cylindrique (et de préférence tronconique en sa base), ladite face intérieure présentant une extrémité inférieure de diamètre $\phi_3$ supérieur à $\phi_2$ et une extrémité supérieure de diamètre $\phi_4$ supérieur à $\phi_3$, l'extrémité supérieure de ladite paroi latérale communiquant avec l'atmosphère et l'extrémité inférieure communiquant avec ladite cavité,

    - un manchon disposé dans le prolongement de ladite partie inférieure de l'embout et délimitant une troisième cavité prolongeant la première cavité de ladite partie inférieure,

  ledit brûleur étant caractérisé en ce que ledit matériau poreux est obtenu à partir d'une composition comprenant en pourcentage du poids total de ladite composition, entre 0 et 5% d'au moins un composé conducteur thermique, de conductivité thermique supérieure à 60 watts par mètre-kelvin, entre 30 et 70% d'au moins un composé réfractaire, entre 2 et 30 % d'au moins un liant, et entre 5 et 40 % d'au moins un agent porogène, et
  en ce que l'extrémité inférieure de ladite face intérieure se termine par un lamage de diamètre $\phi_3$, communiquant avec ladite première cavité (2).

Par lamage, on entend, au sens de la présente invention, un usinage cylindrique réalisé autour d'un perçage communiquant avec la première cavité de la partie inférieure de l'embout

Avec une telle composition, le matériau poreux du bruleur selon l'invention présente une porosité comprise entre 57% et 63%, et de préférence de l'ordre de 59%, et des interconnexions entre 8 et 11 $\mu$m.

Par interconnexions, on entend, au sens de la présente invention le diamètre médian des interconnexions poreuses.

Grâce à la présence du lamage ainsi qu'à la nature et à la porosité du matériau poreux constitutif du brûleur, il est possible d'atteindre de bonnes performances en fonctionnement, comme illustré dans les exemples ci-après, et ce, quelle que soit la longueur du manchon (ou fût), qui peut être aussi faible que 12 mm, ce qui n'était auparavant pas envisageable, notamment avec le brûleur enseigné dans la demande de brevet français du demandeur FR2905164.

De manière avantageuse, la profondeur P, qui tient compte du lamage de la première cavité, peut être comprise entre 2 et 8 mm, de préférence entre 5 et 7,5 mm et mieux de l'ordre de 7 mm.

De manière avantageuse, le diamètre extérieur $\phi_1$ de la partie inférieure de l'embout peut être compris entre 14 et 17 mm, de préférence entre 15 et 16 mm, et mieux de l'ordre de 15,6 mm.

De manière avantageuse, le brûleur selon l'invention peut présenter un épaulement périphérique séparant la partie inférieure de l'embout du manchon.

De manière avantageuse, le matériau poreux est obtenu à partir d'une composition comprenant en pourcentage du poids total de ladite composition, entre 0,5 et 2% dudit composé conducteur thermique, entre 40 et 70% dudit composé réfractaire, entre 5 et 30 % dudit liant, et entre 8 et 40 % d'au moins un agent porogène. La composition du matériau poreux selon l'invention comprend notamment un composé conducteur thermique et un composé réfractaire.

A titre de composé conducteur thermique utilisable dans le matériau poreux du brûleur selon l'invention, on pourra avantageusement utiliser du carbure de silicium, de préférence présent à raison de 1% en poids par rapport au poids total de ladite composition. Le pourcentage de composé conducteur thermique (entre 0 et 5%, et de préférence de l'ordre de 1%) a été optimisé de manière à obtenir des caractéristiques de fonctionnement idéales.

A titre de composé réfractaire, on utilisera de préférence un composé réfractaire présentant une conductivité thermique inférieure à la conductivité thermique dudit conducteur thermique. De manière avantageuse, ledit composé réfractaire peut être choisi dans le groupe constitué de l'alumine, la silice, la mullite, la zircone, la cordiérite, et leurs mélanges. Le matériau réfractaire est de préférence la mullite. La mullite peut par exemple être remplacée par ou mélangée à l'alumine, la silice, la zircone, la cordiérite, ou un de leurs mélanges. La silice est de préférence introduite dans la composition de l'invention en mélange avec un autre composé réfractaire. Le composé réfractaire, en plus de son bon comportement mécanique à haute température, joue un rôle principal d'isolant au sein du matériau poreux obtenu à partir de la composition selon l'invention.

Outre le composé conducteur thermique et composé réfractaire, la composition du matériau poreux selon l'invention comprend un liant et un agent porogène.

Par liant, on entend au sens de la présente invention un composé minéral permettant un frittage à une température inférieure ou égale à 1100 °C.

Le liant est de manière avantageuse un verre présentant un pourcentage en silice variable, plus particulièrement un verre ordinaire à base d'oxydes, par exemple incluant environ 70% de silice et environ 30% d'oxydes de calcium et de sodium, ou un verre spécial incluant des oxydes d'éléments divers tels que bore ou phosphate, par exemple un verre borosilicaté.

Le liant améliore l'aptitude à la mise en forme, apporte la cohésion mécanique en cru d'une pièce et permet d'obtenir un matériau poreux et résistant mécaniquement. Le liant est choisi en particulier parmi les composés de frittage à basse température, c'est à dire les composés permettant le frittage de la composition dans laquelle ils interviennent à cette température, inférieure ou égale à 1100°C. Par exemple, lors de la montée en température lors du frittage de la composition de l'invention, le verre se ramollit et mouille les particules de composé conducteur thermique. Une phase vitreuse est ainsi obtenue. Lors, ensuite, de la descente en température, les particules sont collées entre elles lors de la solidification de la phase vitreuse.

A titre d'agent porogène utilisable dans le matériau poreux du bruleur selon l'invention, on pourra avantageusement choisir tout composé porogène (naturel ou de synthèse), dont la granulométrie est maîtrisée et contrôlée, notamment au cours de sa fabrication et de son stockage. Par exemple, on pourra avantageusement utiliser à titre d'agent porogène le polyméthacrylate de méthyle (PMMA). Celui si pourra avantageusement être présent à raison de 21% en poids par rapport au poids total de ladite composition. L'agent porogène permet la formation de pores au cours du frittage de la composition selon l'invention.

Ainsi, de manière préférentielle, le matériau poreux pourra être réalisé à partir d'une composition comprenant en pourcentage du poids total de ladite composition, de l'ordre de 1% de carbure de silicium, entre 60 et 70 % de mullite, entre 5 et 15 % de verre, et entre 18 et 30% de PMMA.

Avec une composition, le matériau poreux du brûleur selon l'invention présente une porosité de l'ordre de 60%, et des interconnexions de l'ordre de 9,5 $\mu$m.

De manière avantageuse, ledit manchon du bruleur peut présenter une longueur comprise entre 12 et 16 mm, et de préférence de l'ordre de 14 mm. Comme illustré dans les exemples, il est possible d'atteindre de bonnes performances avec un brûleur selon l'invention présentant une longueur de manchon pouvant être aussi faible que 13 mm.

La présente invention a encore pour objet un flacon à combustion catalytique, adapté à contenir un liquide combustible et à recevoir au niveau de son goulot un brûleur à combustion catalytique recevant une mèche trempant dans ledit liquide, caractérisé en ce que ledit flacon est équipé d'un brûleur selon l'invention tel que défini précédemment.

D'autres caractéristiques et avantages de l'invention ressortiront clairement de la description détaillée qui en est faite ci-après, à titre indicatif et nullement limitatif, en référence aux dessins annexés dans lesquels :

- la figure 1 représente schématiquement une coupe transversale d'un exemple de brûleur selon l'invention ;

- la figure 2 représente une vue en perspective du brûleur illustré sur la figure 1 ;

- la figure 3 représente schématiquement une coupe transversale d'un exemple de brûleur connu de l'art antérieur ;

- la figure 4 est une vue schématique en élévation d'un flacon équipé de l'un des brûleurs à combustion représentés sur les figures 1 et 2 d'une part et sur la figure 3 d'autre part.

Les caractéristiques techniques communes à ces deux figures sont désignées chacune par la même référence numérique dans les figures concernées.

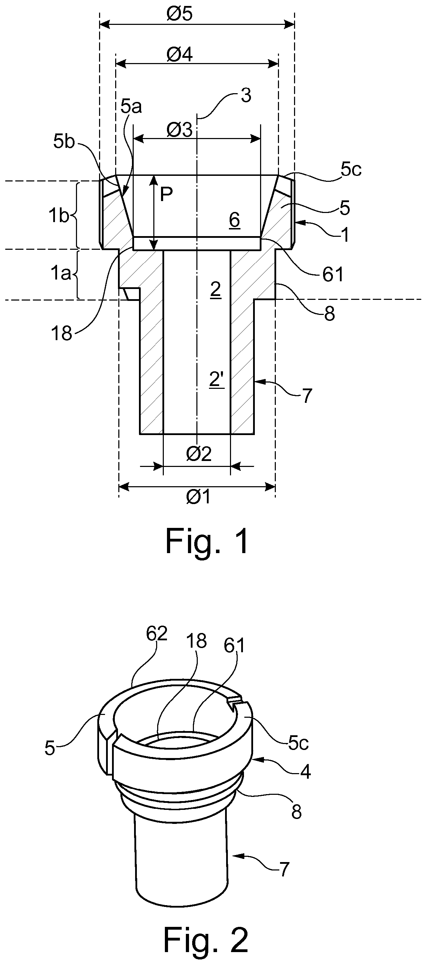

Sur les figures 1 et 2, est représenté schématiquement un exemple de brûleur 10 selon l'invention, en coupe transversale et en perspective latérale respectivement. Ces deux figures montrent notamment que cet exemple de brûleur 10 selon l'invention comprend d'une part un embout 1 avec une partie inférieure 1a et une partie supérieure 1b, et d'autre part un manchon 7 disposé dans le prolongement de la de partie inférieure 1a de l'embout 1.

En ce qui concerne plus particulièrement l'embout 1, celui-ci comporte :

- la partie inférieure 1a de diamètre extérieur $\phi_1$ et délimitant une première cavité 2 de diamètre $\phi_2$, cette première cavité 2, qui s'étend le long d'un axe principal 3 est adaptée à recevoir une mèche propre à imbiber l'embout 1 d'une composition combustible, et
- une partie supérieure 1b présentant une paroi périphérique latérale 5 comportant une face intérieure 5a de forme essentiellement tronconique et délimitant une deuxième cavité 6 de profondeur P, une face extérieure 5b de forme cylindrique et de diamètre $\phi_5$, et une face supérieure 5c de forme annulaire et légèrement tronconique en sa base.

La face intérieure 5a présente une extrémité inférieure 61 de diamètre $\phi_3$ supérieur à $\phi_2$ et une extrémité supérieure 62 de diamètre $\phi_4$ supérieur à $\phi_3$, l'extrémité supérieure 62 de la paroi latérale 5 communiquant avec l'atmosphère et l'extrémité inférieure 61 communiquant avec ladite première cavité 2. Les première 2 et deuxième 6 cavités sont reliées. L'extrémité inférieure 61 de la face intérieure 5a se termine par un lamage 18 de diamètre $\phi_3$, communiquant avec la cavité 2.

Le brûleur selon l'invention peut en outre présenter un épaulement périphérique 8 séparant la partie inférieure 1a de l'embout 1 du manchon 7.

En ce qui concerne plus particulièrement le manchon (également appelé fût) 7, celui-ci est disposé dans le prolongement de la partie inférieure 1a de l'embout 1. Il délimite une troisième cavité 2' prolongeant la première cavité 2 de la partie inférieure 1a. Ce manchon est constitué du même matériau poreux que l'embout 1.

Sur la figure 3, est représentée schématiquement une coupe transversale d'un exemple de brûleur 10 connu de l'art antérieur. Le brûleur 10 de la figure 3 est sensiblement identique à celui représenté sur les figures 1 et 2, sauf en ce qui concerne le lamage : le brûleur de la figure 3 n'en comporte pas, de sorte que la face intérieure 5a est entièrement tronconique entre ses deux extrémités 61 et 62. En outre, les diamètres $\phi_1$, $\phi_3$ , et $\phi_5$ sont également différents.

Afin de tester le fonctionnement catalytique des bruleurs représentés sur les figures 1 à 3, ceux-ci ont été disposés dans un flacon 20 à combustion catalytique, représenté sur la figure 4. Un tel flacon 20 contient en fonctionnement un liquide combustible 30. Le brûleur 10 (soit celui selon l'invention tel que représenté sur les figures 1 et 2, soit celui de l'art antérieur représenté sur la figure 3) est installé dans le goulot 50 du flacon (par exemple à l'aide d'une embase métallique placée dans le goulot 50). Une mèche 40 est reçue à l'intérieur des première 2 et deuxième 24 cavités du brûleur 10, cette mèche 40 à combustion catalytique recevant une mèche (40) trempant dans le liquide 30. Le flacon 20 peut être un flacon de forme quelconque présentant un goulot 50 dans lequel est adapté le brûleur 10. Le liquide combustible 30 est habituellement un alcool, par exemple de l'alcool isopropylique, ou tout autre liquide combustible approprié compatible avec la législation en vigueur dans ce domaine. En particulier, ce combustible doit être tel que sa vaporisation et sa combustion catalytique ne dégagent pas d'odeur désagréable. Le liquide combustible 30 peut en outre comprendre une matière parfumée et/ou une matière active. La mèche 40 est une mèche connue quelconque, par exemple une mèche en coton, ou une mèche en matière minérale, par exemple en fibres minérales. Les exemples suivants illustrent l'invention, en liaison avec les figures, sans toutefois en limiter la portée.

Dans ces exemples, sauf indication contraire, tous les pourcentages et parties sont exprimés en pourcentages massiques.

EXEMPLES

Composés entrant dans la composition des matériaux poreux utilisés :

- composé conducteur thermique : carbure de silicium,

- composé réfractaire : mullite,

- liant : verre,

- agent porogène : polyméthacrylate de méthyle (PMMA) .

Compositions :

Les brûleurs utilisés dans les exemples sont réalisés par pressage à sec à partir des compositions suivantes C1 et C2 indiquées dans le tableau 1. Pour chacune de ces compositions, nous avons indiqué dans le tableau 1 la porosité de la structure céramique et le diamètre médian des interconnexions.

Tableau 1

| Composition | Mullite (%) | SiC (%) | Verre (%) | PMMA (%) | Porosité (%) | Diamètre d'interconnexions ($\mu$m) |
|---|---|---|---|---|---|---|
| C1 | 64 | 5 | 10 | 21 | 58 à 60% à 975°C | 9 |
| C2 | 66,5 | 1 | 11,5 | 21 | 60,2 à 1050°C | 9,5 |

**Brûleurs utilisés :**

- Pour les exemples comparatifs :

  ◦ brûleur 1C représenté sur la figure 3, dont la face supérieure 5c est munie/imprégnée d'un catalyseur, et constitué d'un matériau poreux obtenu à partir de la composition C1,
  ◦ brûleur 2C identique au brûleur 1C mais exempt de catalyseur (représenté sur la figure 3), et constitué d'un matériau poreux obtenu à partir de la composition C1.

- Pour les exemples selon l'invention :

  ◦ brûleur 1 représenté sur les figures 1 et 2, dont la face supérieure 5c est munie/imprégnée d'un catalyseur, et constitué d'un matériau poreux obtenu à partir de la composition C2,
  ◦ brûleur 2 identique au brûleur 1 mais exempt de catalyseur (représenté sur les figures 1 et 2). et constitué d'un matériau poreux obtenu à partir de la composition C2.

Le catalyseur utilisé est par exemple tel que celui décrit dans la demande de brevet EP1084267 appartenant à la demanderesse : un métal appartenant au groupe 9 ou 10 du tableau de classification périodique des éléments (selon la terminologie recommandée par l'UICPA).

En fonctionnement, lorsque le brûleur est muni d'un catalyseur dans sa partie périphérique annulaire 5c (visible sur la figure 2), la partie du liquide combustible qui parvient dans cette partie 5c y subit une combustion catalytique qui maintient cette partie à une température élevée.

**Flacon utilisé :** celui représenté sur la figure 4 pour les brûleurs représentés sur les figures 1 et 2 d'une part et sur la figure 3 d'autre part.

**Mèche utilisée :** mèche en coton.

**Liquide combustible utilisé :** alcool isopropylique.

**Tests et mesures**

1) Porosité (%) et Diamètre D des interconnexions

On mesure la porosité ouverte du matériau poreux constitutif du brûleur par intrusion de mercure dans le matériau d'un porosimètre de marque MICROMERITICS AUTOPORE IV 9510. Cette mesure est réalisée à une pression maximale de 414 MPa environ, ce qui correspond à une taille minimale des pores détectables de l'ordre de 0,0035 $\mu$m.

Méthode et protocole opératoire :

La mesure de la porosité par intrusion de mercure est basée sur le principe de pénétration d'un liquide non réactif dans un matériau poreux, en immergeant celui-ci dans le liquide et en augmentant la pression de manière isostatique. Le mercure, n'ayant pas de réaction avec la plupart des matériaux, est de plus un liquide idéal du fait de la valeur élevée de son angle de contact, il ne mouille pas la plupart des matériaux.

A partir de cette mesure, on détermine la taille des pores en terme de diamètre D en $\mu$m (diamètre des interconnexions), alors pénétré, est inversement proportionnelle à la pression appliquée, P, d'après l'équation de WASBURN :

$$D = \frac{-4\gamma\cos\Theta}{P}$$

Avec : $\gamma$: tension superficielle du mercure,
$\gamma$= 0,00485 N/cm (485 dynes/cm).
$\theta$ : angle de contact du mercure, $\theta$=140°

2) Caractéristiques de fonctionnement des brûleurs 10 installés sur le flacon 20

En fonctionnement, le liquide combustible 30 du flacon 20 monte dans la mèche 40 par capillarité et pénètre dans les pores de la matière poreuse du brûleur, qui lorsqu'il a été préchauffé, assure sa combustion catalytique. Les brûleurs précités sont dans le cadre des essais réalisés testés pour différents modes de fonctionnement :

• fonctionnement à vide : on laisse fonctionner le brûleur jusqu'à consommation complète du liquide combustible 30 ;

• rallumage: le brûleur est utilisé en continu pendant une durée déterminée constante, puis régulièrement éteint et rallumé sur plusieurs jours.

Ces différents essais sont présentés ci-après, en spécifiant pour chaque configuration, la longueur du manchon 7.

EXEMPLE 1

On compare les performances des brûleurs 2 et 2C (sans catalyseur sur le dessus), dans lequel la mèche est montée en étant enserrée par le manchon).

Les performances en fonctionnement et les caractérisations physico-chimiques du brûleur utilisé sont indiquées dans le tableau 2 ci-après, pour différents types de matériau poreux et de longueurs de manchon 7 pour le brûleur 2.

Tableau 2

| brûleur | porosité (%) | Longueur de manchon 7 (mm) | Rallumage |
|---------|--------------|----------------------------|-----------|
| 2C | 58-60 | 20 | conforme |
| 2 | 57.82-58.3 | 13 | conforme |
| 2 | 59.4 | 17,5 | Conforme |

Le brûleur selon l'invention permet d'atteindre des performances en fonctionnement équivalentes à celle du brûleur connu de l'art antérieur avec une longueur de manchon bien inférieure.

EXEMPLE 2

On compare les performances des brûleurs 2 et 2C (sans catalyseur sur le dessus), dans lequel la mèche est montée directement dans le manchon, sans être enserrée).

Les performances en fonctionnement et les caractérisations physico-chimiques du brûleur utilisé sont indiquées dans le tableau 3 ci-après, pour différents types de matériau poreux et de longueurs de manchon 7 pour le brûleur.

Tableau 3

| brûleur | porosité (%) | Longueur de manchon 7 (mm) | Fonctionnement à vide |
|---|---|---|---|
| 2C | 58-60 | 20 | non conforme |
| 2 | 57,82-58,3 | 13 | conforme |
| 2 | 58.91-59.2 | 13 | conforme |

Le brûleur selon l'invention permet d'atteindre de bonnes performances en fonctionnement et ce, avec une longueur de manchon bien inférieure, contrairement au brûleur de l'art antérieur avec une longueur de manchon bien supérieure.

EXEMPLE 3

On compare les performances des brûleurs 1 et 1C (avec catalyseur sur le dessus), dans lequel la mèche est montée en étant enserrée par le manchon).

Les performances en fonctionnement et les caractérisations physico-chimiques du brûleur utilisé sont indiquées dans le tableau 3 ci-après, pour différents types de matériau poreux et de longueurs de manchon 7 pour le brûleur.

Tableau 3

| brûleur | porosité (%) | Longueur de manchon 7 (mm) | Fonctionnement à vide |
|---|---|---|---|
| 1C | 58-60 | 20 | conforme |
| 1 | 57,82-58,3 | 13 | conforme |
| 1 | 58,91-59,2 | 13 | Conforme |

Le brûleur selon l'invention permet d'atteindre des performances en fonctionnement équivalentes à celle du brûleur connu de l'art antérieur avec une longueur de manchon bien inférieure.

**Revendications**

1. Brûleur (10) à combustion catalytique en matériau poreux comprenant :

   - un embout (1) comportant :

      • une partie inférieure (1a) de diamètre extérieur $\phi_1$ et délimitant une première cavité (2) de diamètre $\phi_2$, ladite première cavité (2) s'étendant le long d'un axe principal (3) et étant adaptée à recevoir une mèche propre à imbiber l'embout (1) d'une composition combustible, et
      • une partie supérieure (1b) présentant une paroi périphérique latérale (5) comportant une face intérieure (5a) de forme essentiellement tronconique et délimitant une deuxième cavité (6) de profondeur P et une face extérieure (5b) de forme essentiellement cylindrique, ladite face intérieure (5a) présentant une extrémité inférieure (61) de diamètre $\phi_3$ supérieur à $\phi_2$ et une extrémité supérieure (62) de diamètre $\phi_4$ supérieur à $\phi_3$, l'extrémité supérieure (61) de ladite paroi latérale (5) communiquant avec l'atmosphère et l'extrémité inférieure (62) communiquant avec ladite première cavité (2),

   - un manchon (7) disposé dans le prolongement de ladite partie inférieure (1a) de l'embout (1) et délimitant une troisième cavité (2') prolongeant ladite première cavité (2) de ladite partie inférieure (1a),

ledit brûleur étant caractérisé en ce ledit matériau poreux est obtenu à partir d'une composition comprenant en pourcentage du poids total de ladite composition, entre 0 et 5% d'au moins un composé conducteur thermique, de conductivité thermique supérieure à 60 watts par mètre-kelvin, entre 30 et 70% d'au moins un composé réfractaire, entre 2 et 30 % d'au moins un liant, et entre 5 et 40 % d'au moins un agent porogène, et

en ce que l'extrémité inférieure (61) de ladite face intérieure (5a) se termine par un lamage (18) de diamètre $\phi_3$, communiquant avec ladite cavité (2).

2. Brûleur (10) selon la revendication 1, dans lequel la profondeur P de ladite cavité (6) est comprise entre 2 et 8 mm, de préférence entre 5 et 7,5 mm et mieux de l'ordre de 7 mm.

3. Brûleur (10) selon la revendication 1 ou 2, dans lequel le diamètre extérieur $\phi_1$ de la partie inférieure (1a) de l'embout (1) est compris entre 14 et 17 mm, de préférence entre 15 et 16 mm, et mieux de l'ordre de 15, 6 mm.

4. Brûleur (10) selon l'une quelconque des revendications 1 à 3, selon lequel ledit matériau poreux est obtenu à partir d'une composition comprenant en pourcentage du poids total de ladite composition, entre 0,5 et 2% dudit composé conducteur thermique, entre 40 et 65 % dudit composé réfractaire, entre 5 et 30 % dudit liant, et entre 8 et 40 % d'au moins un agent porogène.

5. Brûleur (10) selon la revendication 4, dans lequel le composé conducteur thermique est du carbure de silicium, de préférence présent à raison de 1% en poids par rapport au poids total de ladite composition.

6. Brûleur (10) selon l'une quelconque des revendications 1 à 5, dans lequel chaque ledit composé réfractaire est choisi dans le groupe constitué de l'alumine, la silice, la mullite, la zircone, la cordiérite, et leurs mélanges, et de préférence la mullite

7. Brûleur (10) selon l'une quelconque des revendications 1 à 6, dans lequel ledit liant est un composé minéral permettant un frittage à une température inférieure ou égale à 1100 °C, de préférence un verre, et mieux un verre borosilicaté.

8. Brûleur (10) selon l'une quelconque des revendications 1 à 7, dans lequel l'agent porogène est le polyméthacrylate de méthyle (PMMA).

9. Brûleur (10) selon la revendication 8, dans lequel le PMMA est présent à raison de 21% en poids par rapport au poids total de ladite composition.

10. Brûleur (10) selon la revendication 9, selon lequel ledit matériau poreux est obtenu à partir d'une composition comprenant en pourcentage du poids total de ladite composition, de l'ordre de 1% de carbure de silicium, entre 60 et 70 % de mullite, entre 5 et 15 % de verre, et entre 18 % et 30% de PMMA.

11. Brûleur (10) selon la revendication 10, dans lequel ledit manchon (7) présente une longueur comprise entre 12 et 16 mm, et de préférence de l'ordre de 14 mm.

12. Flacon (20) à combustion catalytique, adapté à contenir un liquide combustible (30) et à recevoir au niveau de son goulot (50) un brûleur (10) à combustion catalytique recevant une mèche (40) trempant dans ledit liquide (30), **caractérisé en ce que** ledit flacon (20) est équipé d'un brûleur (10) tel que défini selon l'une quelconque des revendications 1 à 11.

**Patentansprüche**

1. Brenner (10) mit katalytischer Verbrennung aus porösem Material, umfassend:

- einen Stutzen (1), umfassend:

• einen unteren Teil (1a), der einen Außendurchmesser $\phi_1$ aufweist und einen ersten Hohlraum (2) mit einem Durchmesser $\phi_2$ begrenzt, wobei sich der erste Hohlraum (2) entlang einer Hauptachse (3) erstreckt und angepasst ist, einen Docht aufzunehmen, der imstande ist, den Stutzen (1) mit einer brennbaren Zusammensetzung zu durchtränken, und

• einen oberen Teil (1b), der eine umlaufende Seitenwand (5) aufweist, die eine Innenfläche (5a) umfasst, die eine im Wesentlichen kegelstumpfförmige Form aufweist und einen zweiten Hohlraum (6) mit einer Tiefe P begrenzt, und eine Außenfläche (5b) mit einer im Wesentlichen zylindrischen Form, wobei die Innenfläche (5a) ein unteres Ende (61) mit einem Durchmesser $\phi_3$ aufweist, der größer ist als $\phi_2$, und ein oberes Ende (62) mit einem Durchmesser $\phi_4$, der größer ist als $\phi_3$, wobei das obere Ende (61) der Seitenwand (5) mit der Atmosphäre kommuniziert und das untere Ende (62) mit dem ersten Hohlraum (2) kommuniziert,

- eine Muffe (7), die in der Verlängerung des unteren Teils (1a) des Stutzens (1) angeordnet ist und einen dritten Hohlraum (2') begrenzt, der den ersten Hohlraum (2) des unteren Teils (1a) verlängert,

wobei der Brenner **dadurch gekennzeichnet ist, dass** das poröse Material ausgehend von einer Zusammensetzung erhalten wird, umfassend, in Prozent vom Gesamtgewicht der Zusammensetzung, zwischen 0 und 5 % mindestens einer Wärmeleiterverbindung, die eine Wärmeleitfähigkeit von über 60 Watt pro Meter und Kelvin aufweist, zwischen 30 und 70 % mindestens einer feuerfesten Verbindung, zwischen 2 und 30 % mindestens eines Bindemittels und zwischen 5 und 40 % mindestens eines Porenbildners, und dadurch, dass das untere Ende (61) der Innenfläche (5a) in einer Senkung (18) mit einem Durchmesser $\phi_3$ endet, die mit dem Hohlraum (2) kommuniziert.

2. Brenner (10) nach Anspruch 1, wobei die Tiefe P des Hohlraums (6) zwischen 2 und 8 mm beträgt, vorzugsweise zwischen 5 und 7,5 mm und besser noch etwa 7 mm.

3. Brenner (10) nach Anspruch 1 oder 2, wobei der Außendurchmesser $\phi_1$ des unteren Teils (1a) des Stutzens (1) zwischen 14 und 17 mm beträgt, vorzugsweise zwischen 15 und 16 mm und besser noch etwa 15,6 mm.

4. Brenner (10) nach einem der Ansprüche 1 bis 3, wobei das poröse Material ausgehend von einer Zusammensetzung erhalten wird, umfassend, in Prozent vom Gesamtgewicht der Zusammensetzung, zwischen 0,5 und 2 % der Wärmeleiterverbindung, zwischen 40 und 65 % der feuerfesten Verbindung, zwischen 5 und 30 % des Bindemittels und zwischen 8 und 40 % mindestens eines Porenbildners.

5. Brenner (10) nach Anspruch 4, wobei die Wärmeleiterverbindung Siliciumkarbid ist, das vorzugsweise zu 1 Gew.-% in Bezug auf das Gesamtgewicht der Zusammensetzung vorhanden ist.

6. Brenner (10) nach einem der Ansprüche 1 bis 5, wobei jede feuerfeste Verbindung ausgewählt wird aus der Gruppe bestehend aus Aluminiumoxid, Siliciumdioxid, Mullit, Zirkonoxid, Cordierit und ihren Gemischen, und vorzugsweise Mullit.

7. Brenner (10) nach einem der Ansprüche 1 bis 6, wobei das Bindemittel eine anorganische Verbindung ist, die eine Sinterung bei einer Temperatur von kleiner oder gleich 1100 °C ermöglicht, vorzugsweise ein Glas, und besser noch ein Borosilikatglas.

8. Brenner (10) nach einem der Ansprüche 1 bis 7, wobei der Porenbildner Polymethylmethacrylat (PMMA) ist.

9. Brenner (10) nach Anspruch 8, wobei das PMMA zu 21 Gew.-% in Bezug auf das Gesamtgewicht der Zusammensetzung vorhanden ist.

10. Brenner (10) nach Anspruch 9, wobei das poröse Material ausgehend von einer Zusammensetzung erhalten wird, umfassend, in Prozent vom Gesamtgewicht der Zusammensetzung, etwa 1 % Siliciumkarbid, zwischen 60 und 70 % Mullit, zwischen 5 und 15 % Glas und zwischen 18 % und 30 % PMMA.

11. Brenner (10) nach Anspruch 10, wobei die Muffe (7) eine Länge aufweist, die zwischen 12 und 16 mm beträgt, und vorzugsweise etwa 14 mm.

12. Flasche (20) mit katalytischer Verbrennung, die angepasst ist, eine brennbare Flüssigkeit (30) zu enthalten und auf Höhe ihres Flaschenhalses (50) einen Brenner (10) mit katalytischer Verbrennung aufzunehmen, der einen Docht (40) aufnimmt, der in der Flüssigkeit (30) eingetaucht ist, **dadurch gekennzeichnet, dass** die Flasche (20) mit einem Brenner (10) ausgestattet ist, so wie er nach einem der Ansprüche 1 bis 11 definiert ist.

**Claims**

1. A catalytic combustion burner (10) made of porous material comprising:

    - a tip (1) including:

        • a lower part (1a) with an outer diameter $\phi1$ and bordering a first cavity (2) with a diameter $\phi2$, said first cavity (2) extending along a main axis (3) adapted to receive a wick intended to soak the tip (1) with a combustible composition, and
        • an upper part (1b) presenting a lateral peripheral wall (5) including an inner surface (5a) with a substantially tapered shape and which borders a second cavity (6) with a depth of P and an outer surface (5b) with a substantially cylindrical shape, said inner surface (5a) presenting a lower end (61) with a diameter $\phi_3$ greater than $\phi2$ and an upper end (62) with a diameter $\phi4$ greater than $\phi3$, the upper end (61) of said lateral wall (5) communicating with the atmosphere and the lower end (62) communicating with said first cavity (2),

    - a sleeve (7) arranged in the extension of said lower part (1a) of the tip (1) and bordering a third cavity (2') extending said first cavity (2) of said lower part (1a),

    said burner being **characterised in that** said porous material is obtained from a composition comprising by percentage of the total weight of said composition, between 0 and 5% of at least one thermally conductive compound, with a thermal conductivity higher than 60 watts per kelvin meter, between 30 and 70% of at least one refractory compound, between 2 and 30% of at least one binder, and between 5 and 40% at least one pore-forming agent, and **in that** the lower end (61) of said inner surface (5a) ends in a counterbore (18) with a diameter of $\phi3$, communicating with said cavity (2).

2. A burner (10) according to claim 1, wherein the depth P of said cavity (6) is comprised between 2 and 8 mm, preferably between 5 and 7.5 mm and best if in the range of 7 mm.

3. A burner (10) according to claim 1 or 2, wherein the outer diameter $\phi1$ of the lower part (1a) of the tip (1) is comprised between 14 and 17 mm, preferably between 15 and 16 mm, and best if in the range of 15.6 mm.

4. A burner (10) according to any one of claims 1 to 3, according to which said porous material is obtained from a composition comprising by percentage of the total weight of said composition, between 0.5 and 2% of said thermally conductive compound, between 40 and 65% of said refractory compound, between 5 and 30% of said binder, and between 8 and 40% at least one pore-forming agent.

5. A burner (10) according to claim 4, wherein the thermally conductive compound is silicon carbide, preferably at 1% by weight relative to the total weight of said composition.

6. A burner (10) according to any of claims 1 to 5, wherein each said refractory compound is selected from among the group made up of aluminium, silica, mullite, zirconia, cordierite, and blends thereof, and preferably mullite.

7. A burner (10) according to any of claims 1 to 6, wherein said binder is a mineral compound that can be sintered at a temperature less than or equal to 1100°C, preferably a glass, and best if a borosilicate glass.

8. A burner (10) according to any of claims 1 to 7, wherein the pore-forming agent is polymethyl methacrylate (PMMA).

9. A burner (10) according to claim 8, wherein PMMA is present at 21% by weight relative to the total weight of said composition.

10. A burner (10) according to claim 9, according to which said porous material is obtained from a composition comprising by percentage of the total weight of said composition, in the range of 1% silicon carbide, between 60 and 70% mullite, between 5 and 15% glass, and between 18% and 30% PMMA.

11. A burner (10) according to claim 10, wherein said sleeve (7) has a length comprised between 12 and 16 mm, and preferably in the range of 14 mm.

12. A catalytic combustion bottle (20), adapted to contain combustible liquid (30) and at the bottleneck (50) thereof receive a catalytic combustion burner (10) receiving a wick (40) soaking in said liquid (30), **characterised in that** said bottle (20) is equipped with a burner (10) as defined in any one of claims 1 to 11.

Fig. 1

Fig. 2

Fig. 3

Fig. 4

**EP 3 563 087 B1**

**Documents brevets cités dans la description**

- FR 2610390
- FR 2905164

- EP 1084267 A